# EUROPEAN PATENT SPECIFICATION

(11) **EP 3 206 680 B1**
(45) Date of publication and mention of the grant of the patent: **21.04.2021**
(21) Application number: 15784113.1
(22) Date of filing: 14.10.2015
(51) Int. Cl.: A61K 31/352, A61P 11/00, A61K 36/185, A61P 25/08

(54) **USE OF CANNABIDIOL IN THE TREATMENT OF NOCTURNAL SNORING IN DOOSE SYNDROME**
VERWENDUNG VON CANNABIDIOL BEI DER BEHANDLUNG VON NÄCHTLICHEM SCHNARCHEN BEI DOOSE-SYNDROME
UTILISATION DE CANNABIDIOL DANS LE TRAITEMENT DES RONFLEMENTS NOCTURNES DANS LA SYNDROME DE DOOSE

(30) Priority: 14.10.2014 GB 201418169
(43) Date of publication of application: 23.08.2017
(73) Proprietor: GW Research Limited, Cambridge, CB24 9BZ (GB)
(72) Inventor: GUY, Geoffrey, Cambridge Cambridgeshire CB24 9BZ (GB); WRIGHT, Stephen, Cambridge Cambridgeshire CB24 9BZ (GB); MEAD, Alice, Cambridge Cambridgeshire CB24 9BZ (GB); DEVINSKY, Orrin, New York, New York 10016 (US)
(74) Representative: HGF
(86) International application number: PCT/GB2015/053030
(87) International publication number: WO 2016/059405

(56) References cited:
- WO-A1-2015/193668
- PORTER BRENDA E ET AL: "Report of a parent survey of cannabidiol-enriched cannabis use in pediatric treatment-resistant epilepsy", EPILEPSY AND BEHAVIOR, vol. 29, no. 3, 1 December 2013 (2013-12-01), pages 574-577, XP028775189, ISSN: 1525-5050, DOI: 10.1016/J.YEBEH.2013.08.037
- Catherine Jacobson ET AL: "Survey of Current Cannabidiol Use in Pediatric Treatment-Resistant Epilepsy", , 22 April 2013 (2013-04-22), XP055238831, Retrieved from the Internet: URL:https://www.thcint.com/uploads/1/9/3/7 /19371199/cannabidiol_use_in_pediatric_epi lepsy.pdf [retrieved on 2016-01-05]

## Description

The present invention relates to the use of cannabidiol (CBD) for the treatment of nocturnal snoring in subjects diagnosed with Doose syndrome.

The CBD is present in the form of a highly purified extract of cannabis which comprises at least 98% (w/w) CBD. Alternatively, the CBD may be a synthetically produced CBD.

### BACKGROUND TO THE INVENTION

Nocturnal snoring is caused by the vibration of the soft tissue in the head and neck as a person breathes in. It can affect many areas of the head and neck including the nasal passages; the soft palate; the base of the tongue; the tonsils and the uvula.

When a person is asleep, the airways in their head and neck relax and narrow. The narrowing of the airways increases the speed in which a person breathes out thus causing changes in the air pressure of the airways. This in turn causes the soft tissue to vibrate by sucking the sides of the airways in.

Evidence suggests that snoring gets worse over time if left untreated. The vibrations that occur during snoring appear to damage blood vessels that supply muscles in the head and neck, which causes the muscles to weaken.

Obstructive sleep apnea (OSA) is a condition where the walls of the throat relax and narrow so much during sleep that normal breathing is interrupted.

Signs of OSA in someone sleeping can include: loud snoring; noisy and laboured breathing; and repeated short periods where breathing is interrupted by gasping or snorting.

Some people with OSA may also experience night sweats and may wake up frequently during the night to urinate. Sufferers of OSA may feel very sleepy during the day and experience poor memory and concentration.

Obstructive sleep apnea can be more common in people with epilepsy. This can be due to the low muscle tone around the airway caused by weight gain associated with side effects of some AED. In addition some AED also have a sedative action.

As well as disrupting sleep, obstructive sleep apnea can trigger seizures.

It is therefore important to address and treat nocturnal snoring in a difficult to treat population such as patients with epilepsy.

Prasad *et al.* (2013) describes a small trial which demonstrated that dronabinol (THC) was able to reduce the apnea hypopnea index in the short term.

US 2004/127572 describes the use of cannabimimetic agents in the treatment of sleep related disorders; WO 2011/063164 describes a slow release formulation which may be useful in treating a range of diseases and conditions including sleep apnea; and WO 2012/068516 describes using as plurality of administrations of low dose cannabinoids to treat sleep apnea.

Porter and Jacobson (2013) describes a parental survey on the use of a cannabidiol enriched extract of a cannabis plant in children with treatment resistant epilepsies. Surveys were collected for 19 children, four of which had Doose syndrome. There is no suggestion in the paper that the treatment of the selected symptom type of nocturnal snoring would be effective with highly purified or synthetic CBD.

Jacobson *et al.* (2013) is an abstract describing the parental survey reported in the paper described above. Again, there is no suggestion that the treatment of the selected symptom type of nocturnal snoring would be effective with highly purified or synthetic CBD.

WO 2015/193668 describes the use of CBD in the treatment of absence seizures in patients with treatment resistant epilepsies which may include Doose syndrome, however there is no suggestion that the use of highly purified or synthetic CBD would be effective in the treatment of nocturnal snoring in subjects diagnosed with Doose syndrome.

The present invention surprisingly demonstrates the ability of highly purified or synthetic CBD to completely eliminate nocturnal snoring in a child with Doose Syndrome.

Doose syndrome is also called myoclonic astatic epilepsy. The long-term outcome for children with Doose Syndrome is highly variable. Some children obtain complete remission and totally normal intellectual development whereas others can become resistant or intractable to AED and can experience mild to severe developmental delay.

Epileptic syndromes such as Doose Syndrome often present with many different types of seizure including tonic-clonic; myoclonic; absence; atonic; myoclonic-absence and tonic seizures. In addition nocturnal snoring is associated with the onset of seizures.

### BRIEF SUMMARY OF THE DISCLOSURE

In accordance with a first aspect of the present invention there is provided cannabidiol (CBD) for use in the treatment of nocturnal snoring in subjects diagnosed with Doose syndrome wherein the CBD is present as a synthetic compound or is in the form of a highly purified extract of cannabis which comprises at least 98% (w/w) CBD.

Preferably the highly purified extract of cannabis comprises less than 0.15% THC. More preferably the highly purified extract of cannabis further comprises up to 1% CBDV.

Preferably the dose of CBD is from 5 to 25 mg/kg/day.

### DEFINITIONS

Definitions of some of the terms used to describe the invention are detailed below:

The cannabinoids described in the present application are listed below along with their standard abbreviations.

**Table 1: Cannabinoids and their abbreviations**

| | | |
|---|---|---|
| CBD | Cannabidiol | 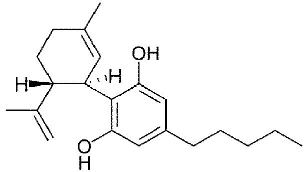 |
| CBDA | Cannabidiolic acid | 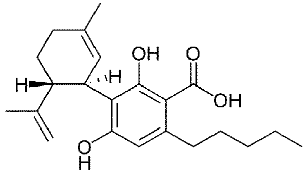 |
| CBDV | Cannabidivarin | 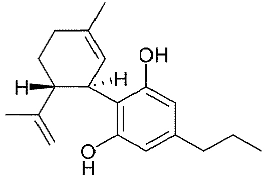 |
| CBDVA | Cannabidivarinic acid | 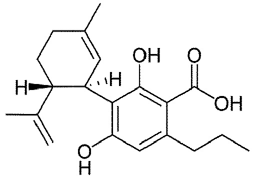 |
| THC | Tetrahydrocannabinol | 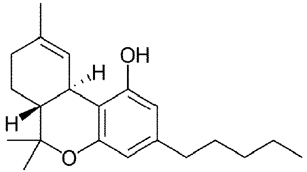 |

The table above is not exhaustive and merely details the cannabinoids which are identified in the present application for reference. So far over 60 different cannabinoids have been identified and these cannabinoids can be split into different groups as follows: Phytocannabinoids; Endocannabinoids and Synthetic cannabinoids (which may be novel cannabinoids or synthetically produced phytocannabinoids or endocannabinoids).

"Phytocannabinoids" are cannabinoids that originate from nature and can be found in the cannabis plant. The phytocannabinoids can be isolated from plants to produce a highly purified extract or can be reproduced synthetically.

"Highly purified cannabinoids" are defined as cannabinoids that have been extracted from the cannabis plant and purified to the extent that other cannabinoids and non-cannabinoid components that are co-extracted with the cannabinoids have been removed, such that the highly purified cannabinoid is greater than or equal to 95% (w/w) pure.

"Synthetic cannabinoids" are compounds that have a cannabinoid or cannabinoid-like structure and are manufactured using chemical means rather than by the plant.

Phytocannabinoids can be obtained as either the neutral (decarboxylated form) or the carboxylic acid form depending on the method used to extract the cannabinoids. For example it is known that heating the carboxylic acid form will cause most of the carboxylic acid form to decarboxylate into the neutral form.

### DETAILED DESCRIPTION

### PREPARATION OF HIGHLY PURIFIED CBD EXTRACT

The following describes the production of the highly-purified (>98% w/w) cannabidiol extract which has a known and constant composition which was used for the expanded access trials described in Examples below.

In summary the drug substance used in the trials is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than 95% CBD w/w, typically greater than 98% w/w.

The *Cannabis sativa* L. plants are grown, harvested, and processed to produce a botanical extract (intermediate) and then purified by crystallization to yield the CBD (drug substance).

The plant starting material is referred to as Botanical Raw Material (BRM); the botanical extract is the intermediate; and the active pharmaceutical ingredient (API) is CBD, the drug substance.

Both the botanical starting material and the botanical extract are controlled by specifications. The drug substance specification is described in Table 2 below.

**Table 2. CBD Specification**

| **Test** | **Test Method** | **Limits** |
|---|---|---|
| Appearance | Visual | Off-white / pale yellow crystals |
| Identification A | HPLC-UV | Retention time of major peak corresponds to certified CBD Reference Standard |
| Identification B | GC-FID/MS | Retention time and mass spectrum of major peak corresponds to certified CBD Reference Standard |
| Identification C | FT-IR | Conforms to reference spectrum for certified CBD Reference Standard |
| Identification D | Melting Point | 65 - 67°C |
| Identification E | Specific Optical Rotation | Conforms with certified CBD Reference Standard; -110° to -140° (in 95% ethanol) |
| Total Purity | Calculation | ≥ 98.0% |
| Chromatographic Purity 1 | HPLC-UV | ≥ 98.0% |
| Chromatographic Purity 2 | GC-FID/MS | ≥ 98.0 % |
| Other Cannabinoids: | HPLC-UV | |
| - CBDA | | NMT 0.15% w/w |
| - CBDV | | NMT 1.0% w/w |
| - Δ⁹ THC | | NMT 0.15% w/w |
| - CBD-C4 | | NMT 0.5% w/w |
| Residual Solvents: | GC | |
| - Alkane | | NMT 0.5% w/w |
| - Ethanol | | NMT 0.5% w/w |
| Residual Water | Karl Fischer | NMT 1.0% w/w |

| | | |
|---|---|---|
| NMT- Not more than | | |

The purity of the CBD drug substance achieved is greater than 98%. The other cannabinoids which may occur in the extract are: CBDA, CBDV, CBD-C4 and THC.

Distinct chemotypes of *Cannabis sativa* L. plant have been produced to maximize the output of the specific chemical constituents, the cannabinoids. One type of plant produces predominantly CBD. Only the (-)-trans isomer occurs naturally, furthermore during purification the stereochemistry of CBD is not affected.

### Production of the Intermediate

An overview of the steps to produce a botanical extract, the intermediate, are as follows:
1. Growing
2. Decarboxylation
3. Extraction No.1 - using liquid CO₂
4. Extraction No.2 - 'winterization' using ethanol
5. Filtration
6. Evaporation

High CBD chemovars were grown, harvested and dried and stored in a dry room until required. The botanical raw material (BRM) was finely chopped using an Apex mill fitted with a 1mm screen. The milled BRM was stored in a freezer for up to 3 months prior to extraction.

Decarboxylation of CBDA to CBD was carried out using a large Heraeus tray oven. The decarboxylation batch size in the Heraeus is approximately 15 Kg. Trays were placed in the oven and heated to 105°C; the BRM took 96.25 minutes to reach 105 °C. Held at 105°C for 15 Minutes. Oven then set to 150°C.; the BRM took 75.7 minutes to reach 150°C; BRM held at 150°C for 130 Minutes. Total time in the oven was 380 Minutes, including 45 minutes cooling and 15 Minutes venting.

Extraction No 1 was performed using liquid CO₂ at 60 bar / 10°C to produce botanical drug substance (BDS) which was used for crystallisation to produce the test material.

The crude CBD BDS was winterised in Extraction No 2 under standard conditions (2 volumes of ethanol at minus 20°C for around 50 hours). The precipitated waxes were removed by filtration and the solvent evaporated using the rotary evaporator (water bath up to 60°C) to yield the BDS.

### Production of the Drug Substance

The manufacturing steps to produce the drug substance from the intermediate botanical extract are as follows:
1. Crystallization using C5-C12 straight chain or branched alkane
2. Filtration
3. Optional recrystallization from C5-C12 straight chain or branched alkane
4. Vacuum drying

Intermediate botanical extract (12kg) produced using the methodology above was dispersed in C5-C12 straight chain or branched alkane (9000 ml, 0.75 vols) in a 30 litre stainless steel vessel.

The mixture was manually agitated to break up any lumps and the sealed container then placed in a freezer for approximately 48 hours.

The crystals were isolated by vacuum filtration, washed with aliquots of cold C5-C12 straight chain or branched alkane (total 12000 ml), and dried under a vacuum of < 10mb at a temperature of 60°C until dry before submitting the drug substance for analysis.

The dried product was stored in a freezer at minus 20°C in a pharmaceutical grade stainless steel container, with FDA food grade approved silicone seal and clamps.

### Production of the Drug Product

The drug product is presented as an oral solution. The oral solution presentation contains 25mg/ml or 100mg/ml CBD, with the excipients sesame oil, ethanol, sucralose and flavouring. Two product strengths are available to allow dose titration across a wide dose range.

The 25 mg/ml solution is appropriate at lower doses and the 100 mg/ml solution at higher doses.

The drug product formulation is as described in Table 3 below:

**Table 3: Drug Product specification**

| **Component** | **Qualitative Composition** | **Function** | **Reference to Quality Standard** |
|---|---|---|---|
| Cannabidiol (CBD) | 25 mg/ml or 100 mg/ml | Active | In-house |
| Anhydrous ethanol | 79.0 mg/ml* | Excipient | Ph.Eur. |
| Sucralose | 0.5 mg/ml | Sweetener | In-house |
| Strawberry flavouring | 0.2 mg/ml | Flavouring | In-house |
| Sesame oil | q.s to 1.0 ml | Excipient | Ph.Eur. |

The drug substance, CBD is insoluble in water. Sesame oil was selected as an excipient to solubilize the drug substance.

A sweetener and fruit flavouring are required to improve palatability of the sesame oil solution.

Ethanol was required to solubilize the sweetener and the flavouring.

The composition can be substantially equivalent, by which is meant the functional ingredients can vary from the qualitative composition specified in Table 6 by an amount of up to 10%.

Example 1 below describes the use of a highly purified cannabis extract comprising cannabidiol (CBD) in an expanded access treatment program in children with TRE.

### EXAMPLE 1: EFFICACY OF CANNABIDIOL REDUCING SEIZURE FREQUENCY IN CHILDREN AND YOUNG ADULTS WITH DOOSE SYNDROME

### Materials and Methods

Of 137 children and young adults with severe, childhood onset treatment-resistant epilepsy (TRE), six suffered from Doose Syndrome. These subjects were tested with a highly purified extract of cannabidiol (CBD) obtained from a cannabis plant. The participants in the study were part of an expanded access compassionate use program for CBD.

All of these patients with a diagnosis of Doose Syndrome presented with multiple seizure types which included tonic-clonic seizures; myoclonic seizures; atonic seizures; absence seizures; myoclonic-absence seizures; and tonic seizures. In addition nocturnal snoring was encountered by one of the patients in the study.

Both patients entered a baseline period of 4 weeks when parents/caregivers kept prospective seizure diaries, noting all countable motor seizure types.

The patients then received a highly purified CBD extract (greater than 98% CBD w/w) in sesame oil, of known and constant composition, at a dose of 5 mg/kg/day in addition to their baseline anti-epileptic drug (AED) regimen.

The daily dose was gradually increased by 2 to 5mg/kg increments until intolerance occurred or a maximum dose of 25 mg/kg/day was achieved.

Patients were seen at regular intervals of 2-4 weeks. Laboratory testing for hematologic, liver, kidney function, and concomitant AED levels was performed at baseline, and after CBD therapy.

All patients were taking at least two concomitant anti-epileptic drugs. These included clobazam; diazepam; lacosamide; lamotrigine; levetiracetam; lorazepam; nordiazepam; n-desmethylclobazam; phenytoin; valproic acid; zonisamide. The average number of concomitant antiepileptic drugs being taken was 2.7. The majority took either clobazam and / or valproic acid.

### Results

There were 6 children and young adult patients who received at least 3 months of treatment all of whom suffered from treatment-resistant epilepsy which is characterised by Doose Syndrome.

The change from baseline in the number of different seizures and total number of seizures after 16 weeks treatment are summarized in Table 4 below.

**Table 4. Changes in Total Seizure Frequency with CBD Therapy**

| **Subject number** | **Change in baseline (%)** | **> 50% reduction in seizures** |
|---|---|---|
| 1 | - 67.4 | Yes |
| 2 | - 100.0 | Yes |
| 3 | - 68.7 | Yes |
| 4 | - 92.0 | Yes |
| 5 | - 100.0 | Yes |
| 6 | 65.8 | No |

Table 4 shows that after 3 months of therapy, there was a decrease in total seizure frequency with five of the Doose syndrome patients.

Table 5 below describes the data that was collated for the other seizure types including nocturnal snoring.

**Table 5. Changes in Total Seizure Frequency with CBD Therapy**

| | **Atonic seizures (n=2)** | **Tonic-clonic seizures (n=5)** | **Absence seizures (n=3)** | **Nocturnal snoring (n=1)** |
|---|---|---|---|---|
| > **50% reduction in seizures** | 50% | 80% | 100% | 100% |
| < **50% reduction in seizures** | 50% | 20% | 0% | 0% |

As can be seen CBD is a very effective treatment for all of the seizure sub-types associated with Doose Syndrome. In addition the fact that it was able to completely eliminate the occurrence of nocturnal snoring is remarkable.

### Conclusions

These data indicate that CBD significantly reduces the number of seizures in a high proportion of patients that do not respond well to existing AED such as those suffering from Doose Syndrome.

It was surprising that in this group of patients which are treatment-resistant that CBD was so effective against, in particular, tonic-clonic seizures, absence seizures and nocturnal snoring.

### References:

Prasad et al. (2013) "Proof of concept trial of dronabinol in obstructive sleep apnea" Front Psychiatry, vol 4, page 1

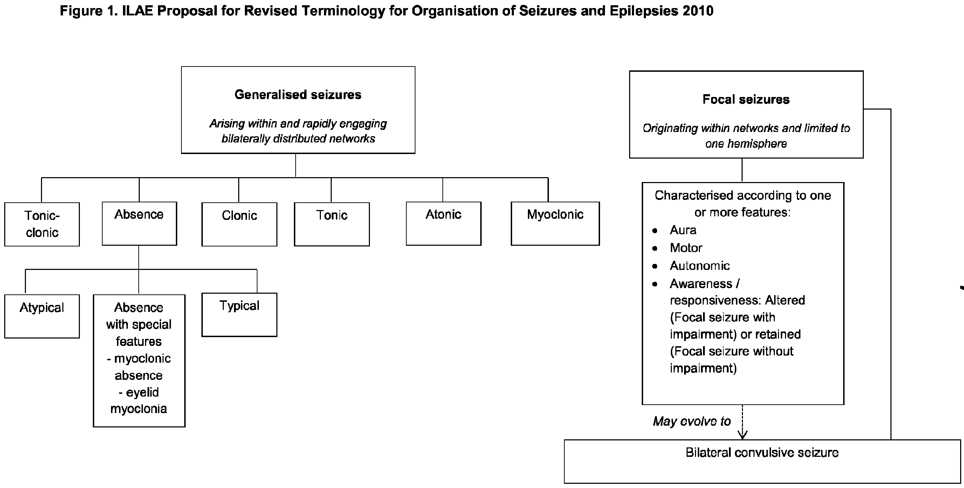

## Claims

1. Cannabidiol (CBD) for use in the treatment of nocturnal snoring in subjects diagnosed with Doose Syndrome wherein the CBD is present as a synthetic compound or is in the form of a highly purified extract of cannabis which comprises at least 98% (w/w) CBD.

2. CBD for use according to any of the preceding claims, wherein the extract comprises less than 0.15% THC.

3. CBD for use according to any of the preceding claims, wherein the extract further comprises up to 1% CBDV.

4. CBD for use according to any of the preceding claims, wherein the dose of CBD is from 5 to 25 mg/kg/day.

## Patentansprüche

1. Cannabidiol (CBD) zur Verwendung in der Behandlung von nächtlichem Schnarchen bei Subjekten, bei denen das Doose-Syndrom diagnostiziert worden ist, wobei das CBD als eine synthetische Verbindung vorhanden ist oder in Form eines hochreinen Cannabisextrakts, das mindestens 98% (w/w) CBD umfasst.

2. CBD zur Verwendung nach einem der vorstehenden Ansprüche, wobei das Extrakt weniger als 0,15% THC umfasst.

3. CBD zur Verwendung nach einem der vorstehenden Ansprüche, wobei das Extrakt ferner bis zu 1 % CBDV umfasst.

4. CBD zur Verwendung nach einem der vorstehenden Ansprüche, wobei die Dosierung von CBD zwischen 5 und 25 mg/kg/Tag beträgt.

## Revendications

1. Cannabidiol (CBD) pour une utilisation dans le traitement de ronflements nocturnes chez des sujets ayant reçu un diagnostic de syndrome de Doose, le CBD étant présent en tant que composé synthétique ou étant sous la forme d'un extrait hautement purifié de cannabis qui comprend au moins 98 % (m/m) de CBD.

2. CBD pour une utilisation selon l'une quelconque des revendications précédentes, l'extrait comprenant moins de 0,15 % de THC.

3. CBD pour une utilisation selon l'une quelconque des revendications précédentes, l'extrait comprenant en outre jusqu'à 1 % de CBDV.

4. CBD pour une utilisation selon l'une quelconque des revendications précédentes, la dose de CBD allant de 5 à 25 mg/kg/jour.
